# EUROPEAN PATENT SPECIFICATION

(11) **EP 0 328 274 B1**
(45) Date of publication and mention of the grant of the patent: **19.10.1994**
(21) Application number: 89300787.2
(22) Date of filing: 27.01.1989
(51) Int. Cl.: C07F 15/00, A61K 31/28

(54) **Pt (IV) complexes**
Pt(IV) Komplexe
Complexes de Pt(IV)

(30) Priority: 02.02.1988 US 151674; 13.01.1989 US 296776
(43) Date of publication of application: 16.08.1989
(73) Proprietor: JOHNSON MATTHEY, INC.,, Malvern Pennsylvania 19355 (US)
(72) Inventor: Abrams, Michael J., Glenmore Pennsylvania 19343 (US); Gaindomenico, Christen M., Exton Pennsylvania 19341 (US); Murrer, Barry A., Reading Berkshire R91 35B (GB); Vollano, Jean F., Exton Pennsylvania 19341 (US)
(74) Representative: Arthur, Bryan Edward

(56) References cited:
- EP-A- 0 237 450
- GB-A- 2 148 891
- US-A- 4 466 924
- JOURNAL OF CLINICAL HEMATOLOGY AND ONCOLOGY, Vol. 7, No. 1, 1977, pages 231-237; Dallas, Texas, US L. M. HALL et al.: "Analogs of sulfato 1,2-diaminocyclohexane platinum (II) (SHP). II. Modifications other than leaving ligand"

## Description

### BACKGROUND OF THE INVENTION

Since the development and use of cis-platin as an effective anti-tumor agent, extensive research has been directed towards finding platinum complexes which might demonstrate similar or better antitumor activity while providing improvements in other properties, e.g., reduced toxicity or other undesired side effects.

A variety of cis-platin analogs have been extensively investigated, including complexes of Pt(IV) such as cis-trans-cis-PtCl₂(OH)₂(i-prNH₂)₂ (U.S. Patent No. 4,394,319) and PtCl₄ (DACH) (U.S. Patent No. 4,550,187). Most Pt antitumor compounds described to date are hydrophilic in that they dissolve more readily in water than they do in common organic solvents, e.g., acetone, methylene chloride, etc. Examples of such hydrophilic complexes include cis-platin itself and carboplatin. Recently, a variety of liposoluble Pt complexes have been reported (e.g., bis(caprato)DACH Pt) that are poorly soluble in water but readily dissolved in organic solvents. See Maeda et al., JPN, J. Cancer Res. 77, 523-525 (1986).

Certain 1-amino-2-aminomethylcyclopentane platinum (II) and (IV) complexes have also been described in U.S. Patent 4,466,924. This patent refers generally to the possibility of including acetate, oxalate, malonate or carboxylate substituents attached to the Pt atom, to which are also attached bis-diamine ligands which form part of a ring system fused to an alicyclic moiety. However, no specific examples of such complexes are given in the patent. Hall et al (Journal of Clinical Haematology and Oncology, vol. 7, no. 1, 1977, pages 231-237) refer generally to a number of analogues of 1, 2-diaminocycloalkane platinum (II) and (IV) compounds, where the platinum (IV) compounds have trans-OH or -Cl groups.

The present invention provides Pt (IV) complexes which demonstrate a high degree of antitumour activity, particularly when orally administered. Many of these complexes exhibit a high degree of solubility in both water and organic solvents and this dual-type solubility property may contribute significantly to the high degree of antitumour activity demonstrated by these complexes when given orally. See J. Blanchard, Am. J. Pharm., 135 (1975) and M. Orme, Br. J. Amaesth., 56, 59 (1984).

The complexes of the invention may be structurally illustrated by Formula I as follows: 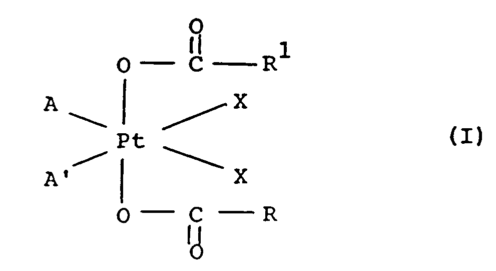
where A and A' are independent groups selected from ammine (that is NH₃), and primary, secondary or tertiary C₁-C₁₀ straight chain, branched chain or cyclic alkylamines, aromatic, heteroaromatic or heterocyclic radicals, or functionalized amines; R and R¹ are selected from the group consisting of hydrogen, C₁-C₁₀ alkyl, alkenyl, aryl, aralky, alkylamino or alkoxy or functionalized derivatives thereof; and X is selected from the group consisting of halogen or alkyl mono- or di-carboxylate.

The A and A' substituents may be the same or different. In the simplest embodiment, both A and A' are NH₃. However, one or both of these substituents may be a C₁ to C₁₀ alkylamine, including straight chain, branched chain or cyclic alkylamines, or an aromatic, heteroaromatic or heterocyclic radical, e.g., anilino, pyridyl, aziridine or morpholine groups, functionalized amines such as NH₂(CH₂)₃CO₂C₂H₅; X¹CO₂-c-C₆H₁₀CH₂NH₂, X¹CO₂CH₂CH₂NH₂ or the like where X¹ is a lower alkyl such as methyl, ethyl, propyl, etc.

As other specific examples of A and/or A', there may be mentioned primary or secondary amines such as n-propylamine (n-C₃H₇NH₂), isopropylamine (i-C₃H₇NH₂), cyclopropylamine (c-C₃H₅NH₂), cyclobutylamine (c-C₄H₇NH₂), isobutylamine (i-C₄H₉NH₂), tertiary butylamine (t-C₄H₉NH₂), neo-pentylamine, tert-amylamine, isoamylamine (i-C₅H₁₁NH₂), cyclopentylamine (c-C₅H₉NH₂), cyclohexylamine (c-C₆H₁₁NH₂), cycloheptylamine (c-C₇H₁₃NH₂) and the corresponding secondary alkylamines, e.g., di-n-propylamine. As will be evident, the alkyl group or groups in the alkyl amine may be straight chain, branched chain or cyclic. Mixed alkyl amine groups are also contemplated.

The R and R¹ substituents may also be the same or different. Representative values for the R and R¹ substituents include alkyl of 1-10 carbons, straight or branched chain such as CH₃, C₂H₅, n-C₃H₇, i-C₃H₇, n-C₄H₉, t-C₄H₉, i-C₄H₉, n-C₅H₁₁, n-C₆H₁₃ or n-C₇H₁₅; cycloalkyl of 3-7 carbons such as cyclopropyl, cyclobutyl, cyclopentyl, cyclohexyl or cycloheptyl; ethenyl, propenyl or other alkenyl; phenyl, tolyl, naphthyl or similar monocyclic or dicyclic aryl; benzyl or phenethyl or like aralkyl; lower alkylamino (primary amino) of up to 6 carbons, e.g., methylamino (CH₃NH-), ethylamino (C₂H₅NH-); and lower alkoxy of 1-6 carbons such as methoxy, ethoxy, etc. R and R¹ may also be a functionalized group such as an alkoxy alkyl (e.g., methoxy-methyl), tosylamino or the like. The definition of R and R¹ as given above for Formula I is intended to include such functionalized or substituted groups.

Representative of the X substituents are Cl, Br or other halogen, or C₁-C₄ alkyl mono-carboxylate such as acetate or di-carboxylate such as malonate or cyclobutane-1,1-dicarboxylate. The X substituents, like the R, R¹ and A/A′ substituents, may be the same or different. They may also be combined in the form of a chelating dicarboxylate radical.

It will be appreciated that the indicated substituents for A, A′, X, R and R¹ may themselves be substituted. For example, when R and/or R¹ are alkyl, the alkyl may be substituted with halogen to provide a chloromethyl substituent.

A preferred subgroup of compounds within Formula (I) are those wherein A is NH₃, A′ is lower alkyl (C₁-C₇)amino, X is chlorine and R and R¹ are lower alkyl (C₁-C₇) or cycloalkyl of 3-7 carbons. Especially preferred are such compounds wherein A′ is cyclopentylamine (c-C₅H₉NH₂) or cyclohexylamine (c-C₆H₁₁NH₂) and R and R¹ are both propyl.

Other subgroups of compounds within Formula (I) include those wherein A is NH₃, A′ is alkylamino, X is chlorine and R and R¹ are lower alkylamino or lower alkoxy.

The compounds of the invention and the starting materials necessary therefor may be prepared by procedures generally known in the art. Starting compounds of the type Pt(OH)₂ Cl₂ AA′ and Pt(OH)₂Cl₂A₂ are conveniently prepared by the reaction of H₂O₂ with the corresponding Pt(II) dichloro species (R.J. Brandon, J.C. Dabrowiak, J. Med. Chem., 27:861 (1984)). The synthesis of the Pt(II) dichloro species is described in the literature (see, M.J. Cleare, J.D. Hoeschele, Plat. Met. Rev., 17:2 (1973) and T.A. Connors et al., Chem. Biol. Interact., 11:145 (1975). For example, the complexes where X is halogen, may be prepared by reacting a compound Pt(OH)₂X₂ A,A′, e.g., Pt(OH)₂Cl₂ (i-PrNH₂)₂, with the appropriate anhydride, isocyanate or pyrocarbonate. The reaction may be carried out by simply mixing the reactants together at room temperature (20-25°C), then chilling to crystallize out the desired product. A generally similar preparation of other Pt complexes of the formula PtCl₂(RCO₂)₂(i-PrNH₂)₂ where R is CF₃, CF₂CF₃ or CF₂CF₂CF₃ has previously been reported but these complexes were found to be practically insoluble in water. See Cowens et al., Int. J. of Mass Spectrometry and Ion Physics, 48, 177-180 (1983).

The complexes wherein X in Formula I is carboxylate may be prepared by acylation of the intermediate formed by hydrogen peroxide oxidation of [Pt(H₂O)₂(R¹NH₂)(R²)²⁺] prepared in turn from PtI₂(R¹NH₂)(R²) and silver nitrate.

As noted, the complexes of the invention demonstrate antitumor activity when administered orally for the treatment of malignant tumors. Accordingly, the complexes may be formulated into tablets, pills, capsules, sterile suspensions, solutions or the like suitable for oral administration. Conventional pharmaceutical carriers, additives, binders, and/or excipients may also be used.

The invention is illustrated, but not limited, by the following examples wherein "c-t-c" is used as an abbreviation for "cis-trans-cis" in order to identify the stereochemistry of ligands around the octahedral Pt center when grouped pairwise in the order written and "c-CₓH_{y}" is used to refer to a cyclic alkyl radical with the ring size specified by the subscript x.

### Example 1

### c-t-c-PtCl₂(O₂CCH₃)₂(NH₃)(c-C₅H₉NH₂) Compound No. 33

0.31 g of c-t-c-PtCl₂ (OH₂)(NH₃)(c-C₅H₉NH₂) was stirred in 7 ml of acetic anhydride until the solid dissolved. The solution was chilled to 5° C and the product crystallized. Addition of ether yielded additional product. The solid was collected, washed with ether and dried in vacuo. The yield was 0.33 g, 87% based on Pt. The product was recrystallized from hot ethyl acetate/hexane.

### Example 2

### c-t-c-PtCl₂(O₂CCH₃)₂(NH₃)(n-C₃H₇NH₂) Compound No. 9

The product was synthesized by the method described in Example 1 but using c-t-c-PtCl₂(OH)₂(NH₃)(n-C₃H₇NH₂) in place of the Pt reactant of Example 1.

### Example 3

### c-t-c-PtCl₂(O₂CCH₂CH₃)₂(NH₃)(n-C₃H₇NH₂) Compound No.10

The product of this example was synthesized using propionic anhydride and 2 g of c-t-c-PtCl₂(OH)₂(n-C₃H₇NH₂)₂. The reaction yielded 0.87 g product (33%).

### Example 4

### c-t-c-PtCl₂(O₂CCH₂CH₃)₂(NH₃)(t-C₄H₉NH₂) Compound No. 23

The method described in Example 1 using propionic anhydride and 1 g of c-t-c-PtCl₂(OH)₂(NH₃)(t-C₄H₉NH₂) was repeated to yield 0.30 g product (23%).

### Example 5

### c-t-c-PtCl₂(O₂CC(CH₃)₃)₂(i-C₃H₇NH₂)₂ Compound No. 6

The product was synthesized by the method described in Example 1 using pivalic anhydride and 1 g of c-t-c-PtCl₂(OH)₂(i-C₃H₇NH₂)₂ and yielding 0.82 g of product (59%).

### Example 6

### c-t-c-PtCl₂(O₂CCH₂CH₂CH₃)₂(i-C₃H₇NH₂)₂ Compound No. 5

The product was synthesized by the method described in the foregoing examples and precipitated out of solution by adding excess hexane and cooling to 0°C. The reactants, butyric anhydride and 1 g of c-t-c-PtCl₂(OH)₂(i-C₃H₇NH₂)₂, yielded 0.85 g product (64%).

### Example 7

### c-t-c-PtCl₂(O₂CC₆H₅)₂(i-C₃H₇NH₂)₂ Compound No. 7

c-t-c-PtCl₂(OH)₂(i-C₃H₇NH₂)₂ (2 g 0.0048 mole) was dissolved in benzoic anhydride (10.82 g), pyridine (0.15 mL) and toluene (5 mL). The product, which precipitated after several hours, was collected, washed with copious amounts of ether to dissolve the excess benzoic anhydride, and dried in vacuo. The product was recrystallized from hot ethyl acetate/hexane. The reaction yielded 1.25 g (41%).

### Example 8

### c-t-c-PtCl₂(O₂CC₂H₅)₂(i-C₃H₇NH₂)₂ Compound No. 4

1 g of c-t-c-PtCl₂(OH)₂(i-C₃H₇NH₂)₂ was suspended in dichloromethane (50 ml) and triethylamine (0.72 g, 3 eq). Propionyl chloride (0.63 ml, 3 eq) in CH₂Cl₂ (5 ml) was added dropwise. After 15 min stirring, water (20 ml) was added, the mixture filtered and the organic layer washed with water (2 x 20 ml), dried over MgSO₄ and evaporated to dryness in vacuo. The residue was recrystallized from ethyl acetate/ether. The yield of c-t-c-PtCl₂(O₂CC₂H₅)₂(i-C₃H₇NH₂)₂ was 0.18 g, 14%.

### Example 9

### c-t-c-PtCl₂(O₂CCH(CH₃)CH₂CH₃)₂NH₃(c-C₆H₁₁NH₂) Compound No. 4

(S)-(+)-2-methylbutyric acid (0.982 g) and triethylamine (0.971 g) were dissolved in dry dimethylacetamide (10 ml) and cooled to 0° C. Isobutylchloroformate (1.3 g) in dimethylacetamide (2 ml) was added dropwise to the solution which was stirred at <4° C for 2 hours c-t-c-PtCl₂(OH)₂NH₃(c-C₆H₁₁NH₂) (1 g) suspended in dimethylacetamide (10 ml) was added and the mixture stirred 24 hours at room temperature. Water (10 ml) and CH₂Cl₂ (10 ml) were added, the organic layer collected and the aqueous layer extracted with further CH₂Cl₂ (10 ml). The combined organics were washed with saturated aqueous NaHCO₃ (2 x 10 ml) and the solvent removed in vacuo. The resulting orange oil was crystallized from ethyl acetate/ether. The yellow crystals (500 mg) of c-t-c-PlCl₂(O₂CCH(CH)₃CH₂CH₃)₂NH₃(c-C₆H₁₁NH₂) were shown by microanalysis to contain 1 mole of dimethylacetamide/Pt. The presence of dimethylacetamide was confirmed by IR spectroscopy.

### Example 10

### c-t-c-PtCl₂(O₂CH)₂NH₃(c-C₆H₁₁NH₂) Compound No. 37

c-t-c-PtCl₂(OH)₂NH₃(c-C₆H₁₁NH₂) (140 mg) was dissolved in formic acid (96%, 2 ml) and heated to 50 °. After 1 hour the solution was cooled to room temperature, the resulting white solid filtered off, washed with water (3 x 5 ml) and dried to give c-t-c-PtCl₂(O₂CH)₂NH₃(c-C₆H₁₁NH₂) (80 mg, 50%).

### Example 11

### c-t-c-PtBr₂(O₂C-n-C₃H₇)₂NH₃(c-C₆H₁₁NH₂) Compound No. 66

cis-PtI₂(NH₃)(c-C₆H₁₁NH₂) (8.01 g) was suspended in a mixture of water (50 ml) and ethanol (10 ml). Silver nitrate (4.7 g, 1.95 eq) was added and the mixture stirred in the dark overnight. The precipitated silver iodide was removed by filtration and HBr (48% aqueous solution, 30 ml) added. After stirring for 1.5 hr the yellow cis-PtBR₂(NH₃)(c-C₆H₁₁NH₂) (6.9 g) was collected by filtration and dried.

The dibromo complex (6.9 g) was oxidized by treatment with H₂O₂ 3 eq/Pt in water (15 ml) and acetone (10 ml) for 3 hr at 60-70° C. On cooling PtBR₂(OH)₂NH₃(c-C₆H₁₁NH₂) crystallized out and was collected by filtration, washed with water and dried. Yield 5.05 g, 67%. This compound (1.5 g) was stirred in butyric anhydride (15 ml) for 3 days at room temperature to give a pale yellow solution which was partitioned between dichloromethane (20 ml) and water (20 ml). The organic layer was washed with saturated aqueous sodium bicarbonate, dried (MgSO₄) and the solvent removed in vacuo. The resulting yellow oil was triturated with hexane to give c-t-c-PtBr₂(O₂C-n-C₃H₇)₂NH₃(c-C₆H₁₁NH₂) (0.39 g, 20%) as a yellow powder.

### Example 12

### c-t-c-PtCl₂(O₂CCH₃)(O₂C-n-CH₃H₇)NH₃(c-C₆H₁₁NH₂) Compound No. 72

3.35 g c-t-c-PtCl₂(OH)₂NH₃(c-C₆H₁₁NH₂) was stirred in a mixture of dichloromethane (15 ml) and diethylether (15 ml). A mixture of butyric anhydride (2.29 g, 1.8 eq/Pt and acetic anhydride (0.164 g, 0.2 eq/Pt) was added and the reaction stirred for 20 hours. The resulting white solid was collected by filtration, washed with ether and dried. The yield was 3.30 g. Thin layer chromatography (TLC) (silica gel, eluting with ethylacetate:dichloromethane, 1:1) showed the solid to be a mixture of two compounds. 0.9g of the above mixture was dissolved in 1:1 ethylacetate:dichloromethane and chromatographed on a silica column. The first compound eluted was c-t-c-PtCl₂(O₂C-n-C₃H₇)₂NH₃(c-C₆H₁₁NH₂) (450 mg). The second compound eluted (50 mg) was the desired product c-t-c-PtCl₂(O₂CCH₃)(O₂C-n-C₃H₇)NH₃(c-C₆H₁₁NH₂) confirmed by microanalysis, NMR and IR spectroscopy. TLC on this sample gave a single spot, Rf 0.3.

### Example 13

### c-t-c-PtCl₂(O₂CCH₃)(O₂C-n-C₄H₉)NH₃(c-C₆H₁₁NH₂) Compound No. 73

c-t-c-PtCl₂(OH)₂(NH₃)(c-C₆H₁₁NH₂) was suspended in a mixture of dichloromethane (10 ml) ether (15 ml), valeric anhydride (1.8 eq/Pt) and acetic anhydride (1.2 eq/Pt) and stirred at room temperature. After 24 hours the solid was collected by filtration, washed with ether and stirred in dichloromethane (20 ml) for 30 min. Unreacted c-t-c-PtCl₂(OH)₂NH₃(c-C₆H₁₁NH₂) (0.65 g) was collected by filtration and the CH₂Cl₂ soluble fraction evaporated in vacuo. The residual oil (0.4 g) was chromatographed on a 7" x 1" diameter column of silica eluting with 1:1 ethylacetate/dichloromethane. Three fractions were collected. The first, c-t-c-PtCl₂(O₂C-n-C₄H₉)₂(NH₃)(c-C₆H₁₁NH₂) (0.092 g) was shown to be identical with a sample of compound 42 prepared by acylation with pure valeric anhydride. The third fraction, c-t-c-PtCl₂(O₂CCH₃)₂(NH₃)(c-C₆H₁₁NH₂) (0.082 g) was identical to an authentic sample of compound 38. The intermediate fraction (0.168 g) was c-t-c-PtCl₂(O₂CCH₃)(O₂C-n-C₄H₉)(NH₃)(c-C₆H₁₁NH₂), confirmed by microanalysis, IR and NMR spectroscopy and was shown to be pure by TLC.

### Example 14

### c-t-c-PtCl₂(O₂CNHCH₃)₂(NH₃)(t-C₄H₉NH₂) Compound No. 26

c-t-c-PtCl₂(OH)₂(NH₃)(t-C₄H₉NH₂) was stirred in n-methyl isocyanate (10 mL/g) for 2 hours. The product was collected, washed with ether and dried in vacuo. 1 g c-t-c-PtCl₂(OH)₂(NH₃)(t-C₄H₉NH₂) starting material yielded 1.26 g product (97%).

### Example 15

### cis-Pt(O₂CCH₃)₄(NH₃)(i-C₃H₇NH₂) Compound No. 68

6.11 g cis-PtI₂(NH₃)(i-C₃H₇NH₂) was added to a stirred solution of AgNO₃ (3.86 g, 1.95 eq.) in water (10 ml). Stirring was continued for 1 hour and charcoal (approximately 200 mg) was added and stirred for 10 min. After filtration, H₂O₂ (30%, 20 ml) was added to the filtrate and the reaction mixture stirred for 72 hours at room temperature. Carbon black (1 g) was added, and the mixture was stirred overnight to remove excess H₂O₂. The carbon was removed by filtration and acetone (200 ml) was added to the filtrate. The reaction mixture was chilled (-20° C) overnight. The resulting pale yellow solid was collected, washed with acetone and ether and dried in vacuo. This solid was added to acetic anhydride (20 ml) and potassium acetate (4 g) and the mixture stirred 24 hours at room temperature and then left one week before being poured into water (100 ml). The mixture was stirred 5 min and then extracted with chloroform (4 x 25 ml). The combined chloroform extracts were washed with saturated aq. NaHCO₃ (2 x 50 ml), saturated aq. NaCl (50 ml) and dried over MgSO₄. On rotary evaporation, some acetic anhydride remained, so ethanol (20 ml) was added, allowed to react for 5 min and removed. This process was repeated until all acetic anhydride was removed as ethyl acetate and acetic acid. The resulting oil was crystallized from ether/hexane yielding yellow needles, 0.57 g, 11% yield.

### Example 16

### c-t-c-Pt-(CBDCA)(O₂CCH₃)₂(NH₃)₂ Compound No. 69

c-Pt(CBDCA)(NH₃)₂ (4.45 g) was heated at 80° for 10 min in a mixture of hydrogen peroxide (30.1, 20 ml). On cooling c-t-c-Pt(CBDCA)(OH)₂(NH₃)₂ crystallized out. The white solid was filtered off, washed with water and dried. The yield was 3.38 g, 70%. c-t-c-Pt(CBDCA)(OH)₂(NH₃)₂ (1 g) was suspended in acetic anhydride (10 ml) and stirred 48 hr at room temperature. Ether (20 ml) was added, and the white solid was collected by filtration, washed with ether, and dried to give c-t-c-Pt(CBDCA)(O₂CCH₃)₂(NH₃)₂ (0.99 g, 82%).
(CBDCA = cyclobutane-1,1-dicarboxylate)

### Example 17

### c-t-c-PtCl₂(O₂COC₂H₅)₂NH₃(NH₂-n-C₃H₇) Compound No. 8

A suspension of 1.25 g of c-t-c-PtCl₂(OH)₂NH₃(NH₂-n-C₃H₇) is stirred in the dark at RT in 15 ml diethylpyrocarbonate until an IR of the solid no longer shows a PtOH stretch at 3520 cm⁻¹ (10 days). The solid product (1.1 g, 63% yield) was collected, washed with ether and dried.

Microanalytical data obtained for compounds representative of the invention are set out in Table 1:

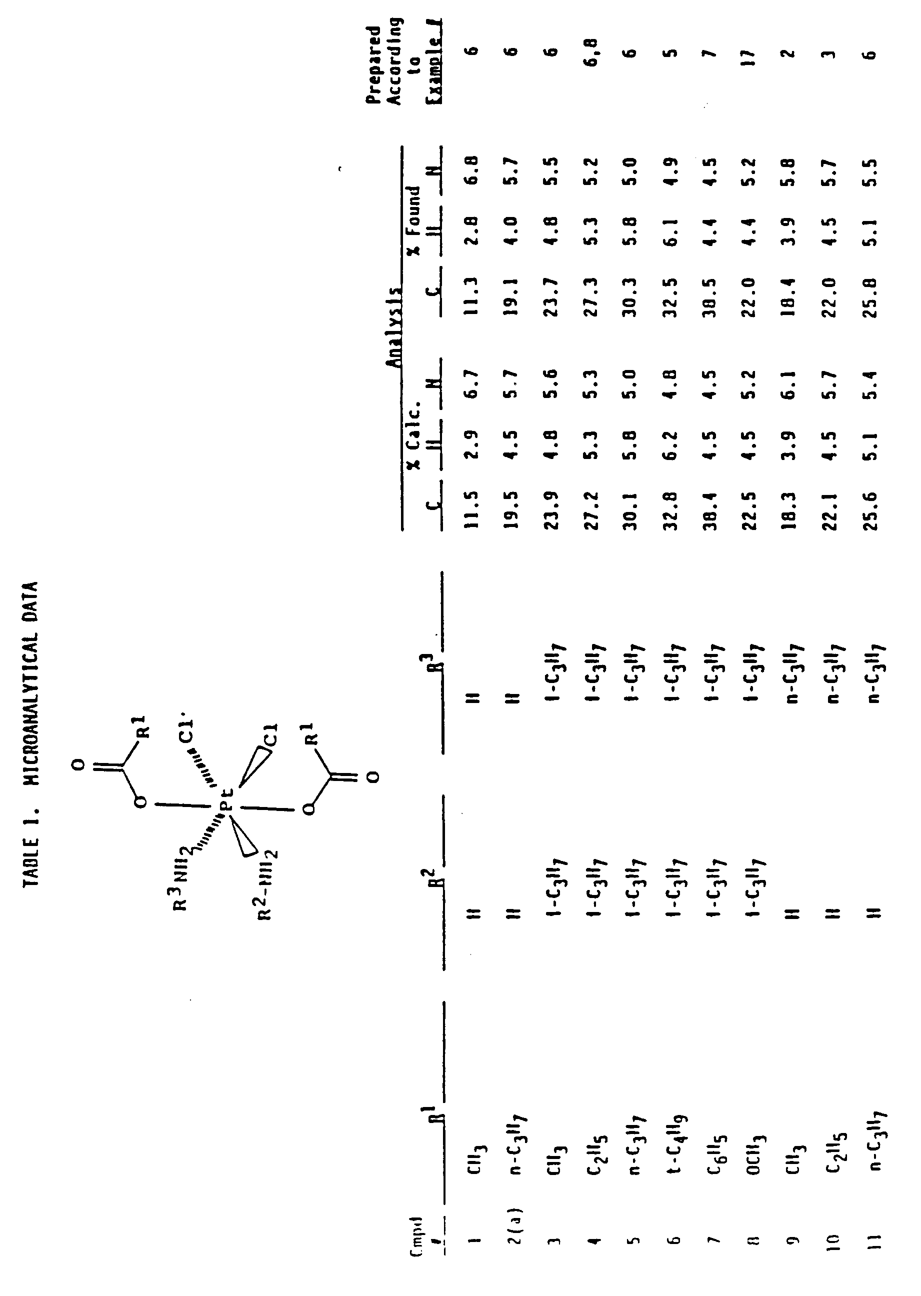

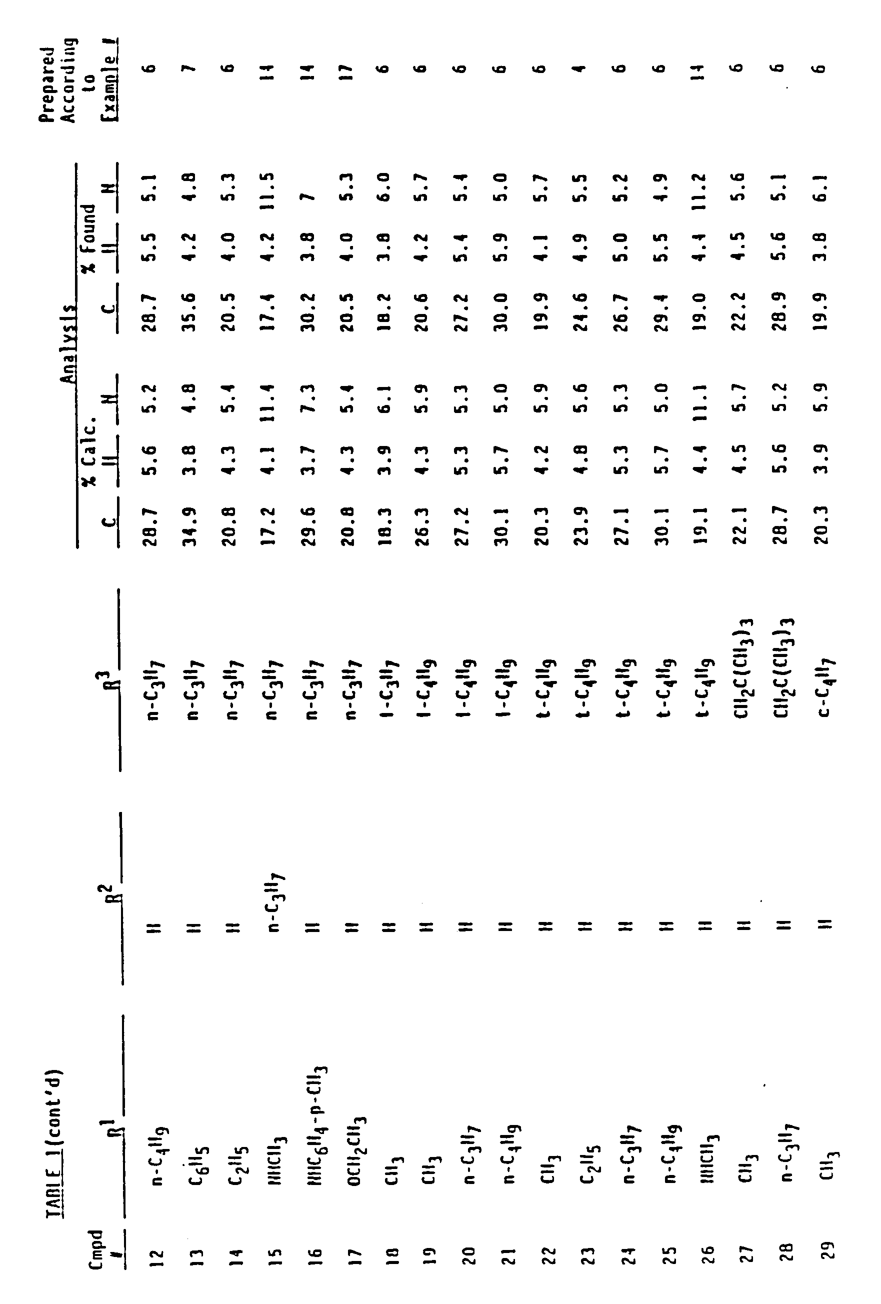

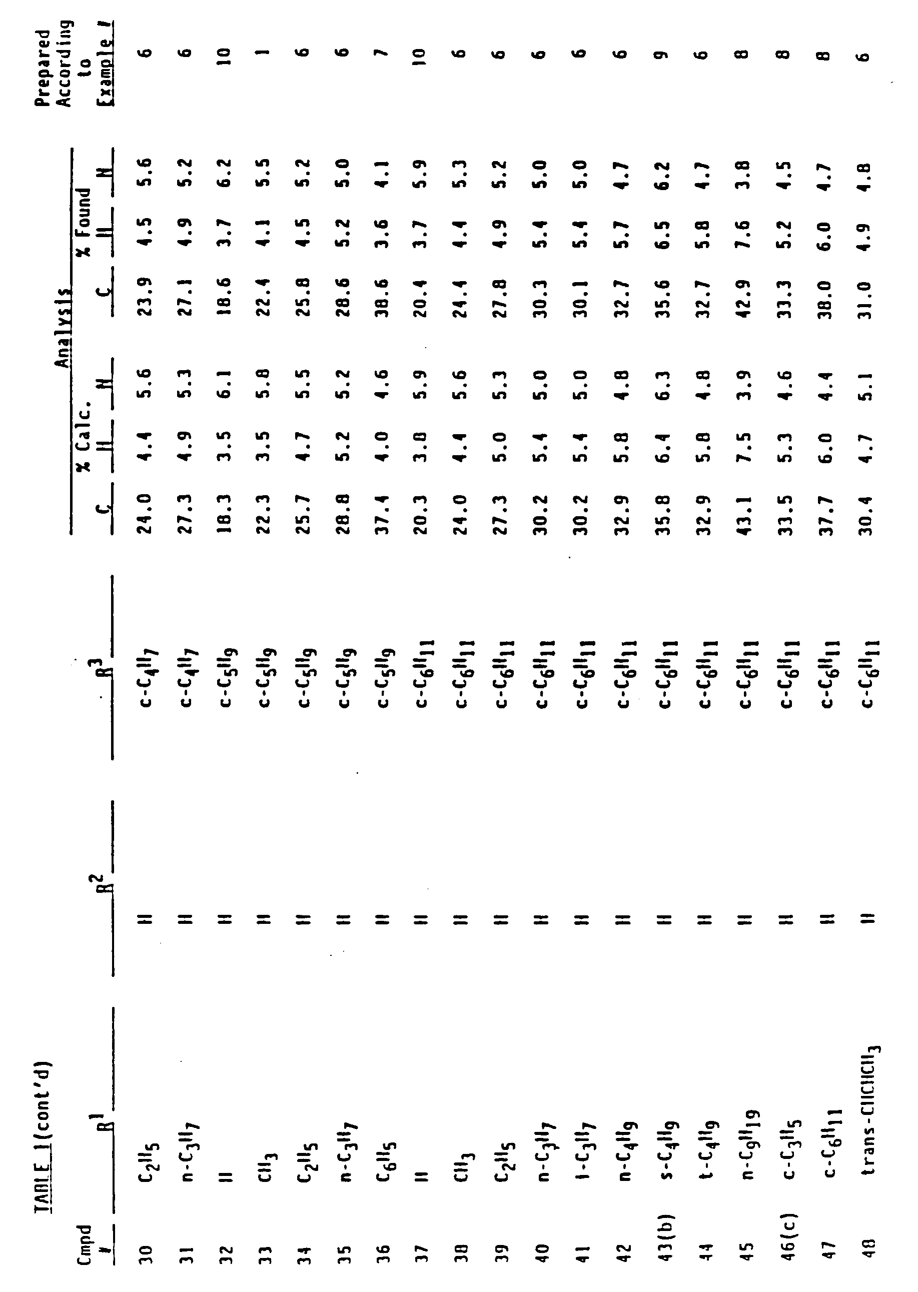

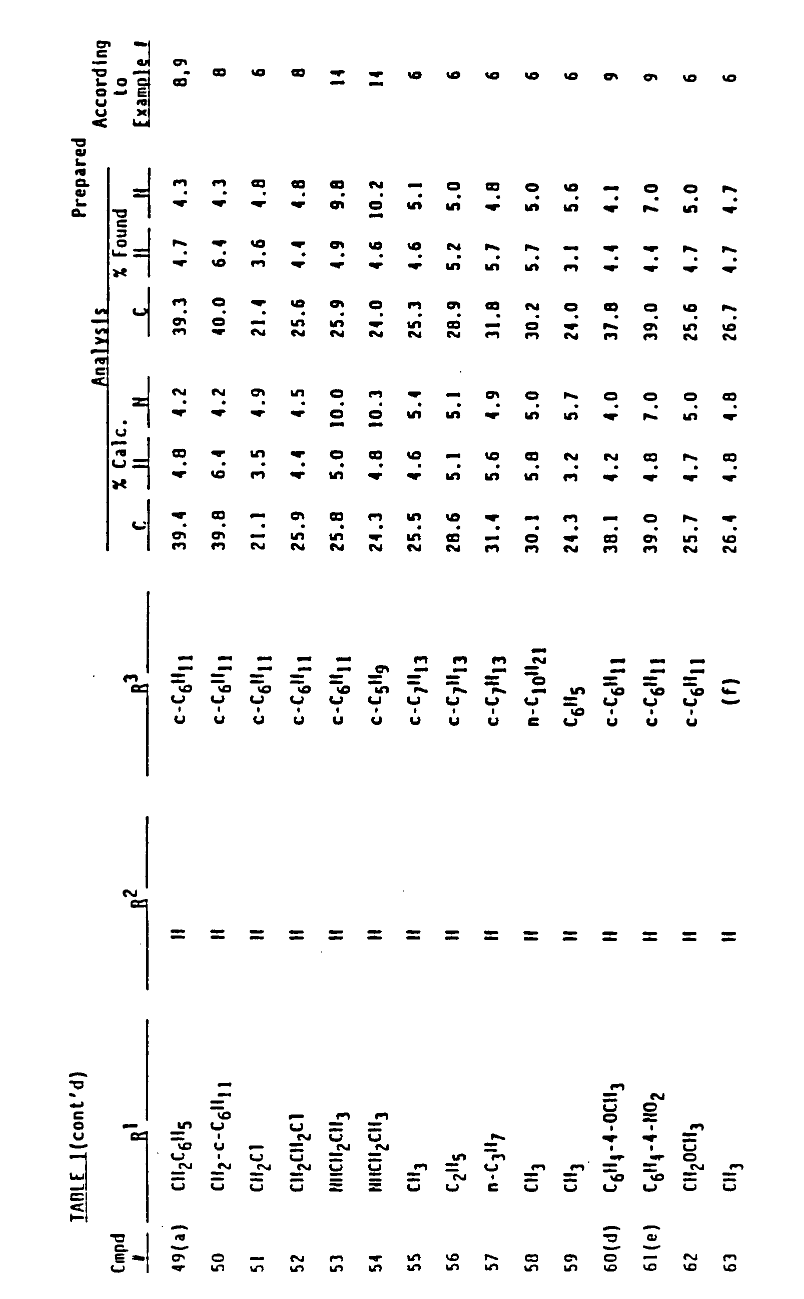

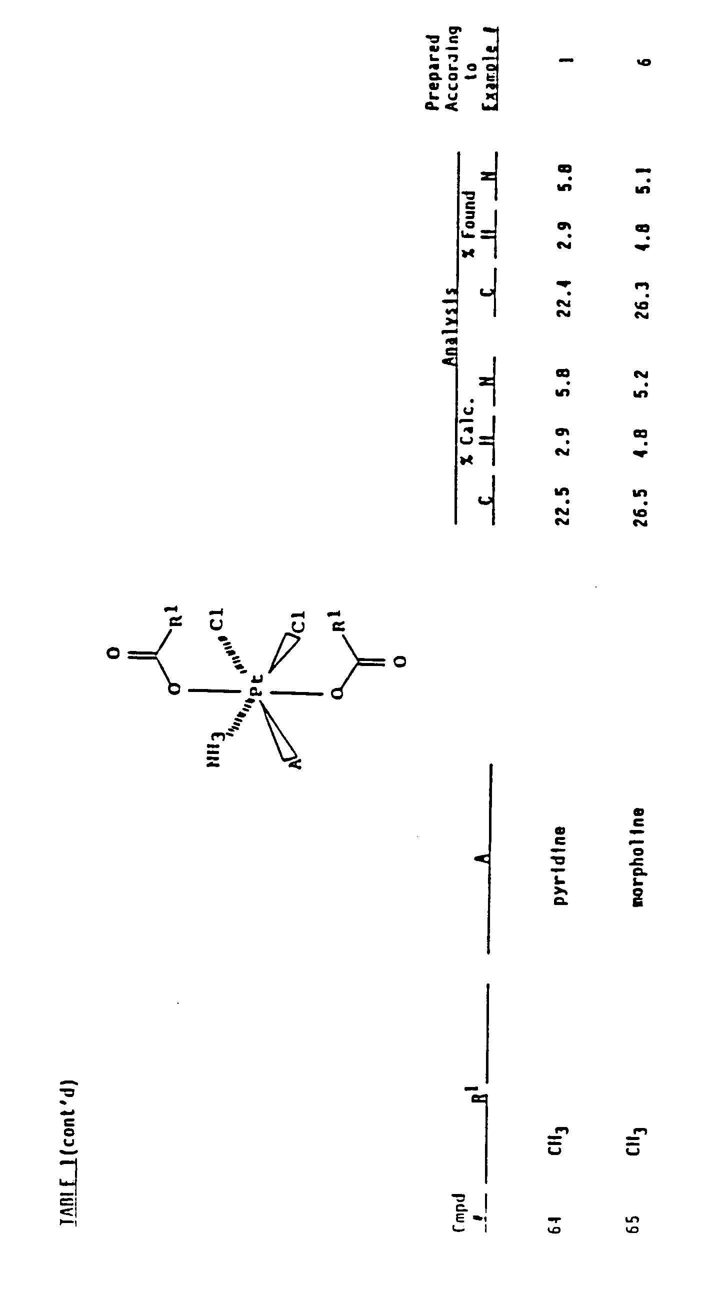

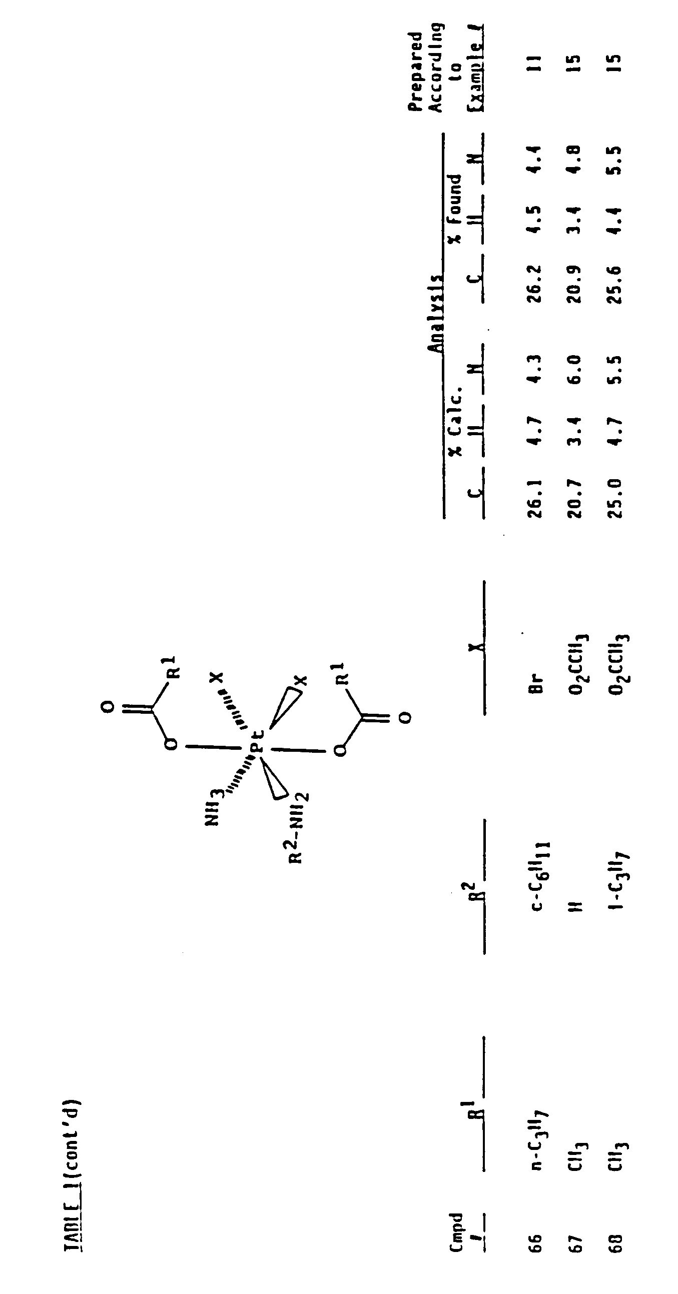

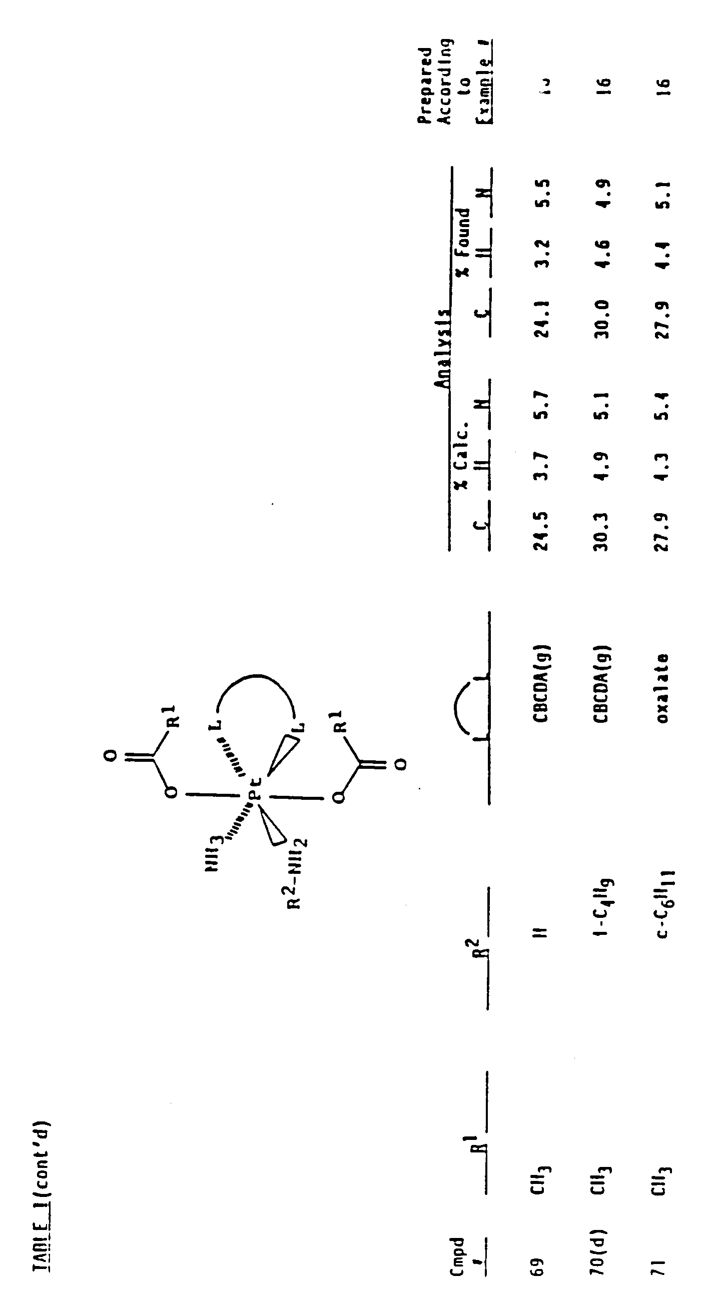

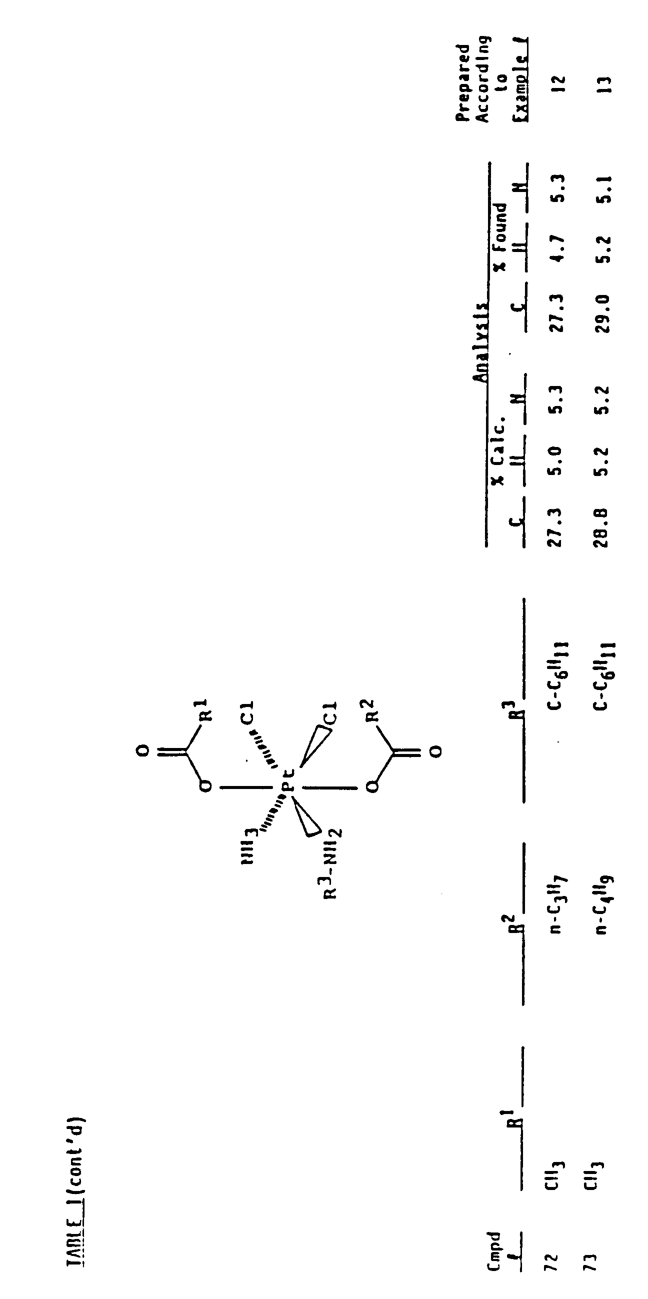

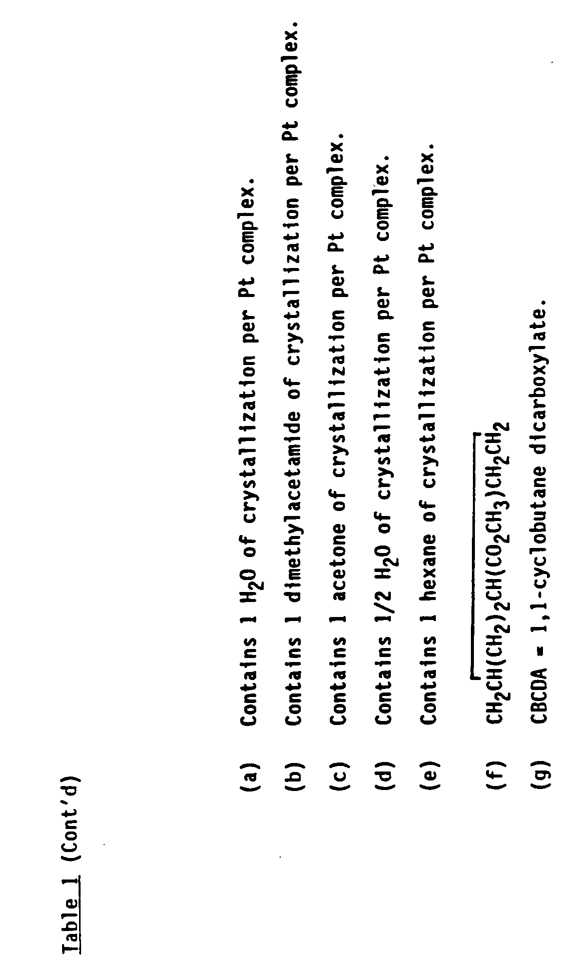

Complexes according to the invention were tested for antitumor activity in a standard test procedure using ADJ/PC6 tumor in mice. The complexes were administered parenterally (I.P.) or orally (P.O.). LD₅₀ and ED₉₀ values in mg/kg were determined with the results shown in Table 2:

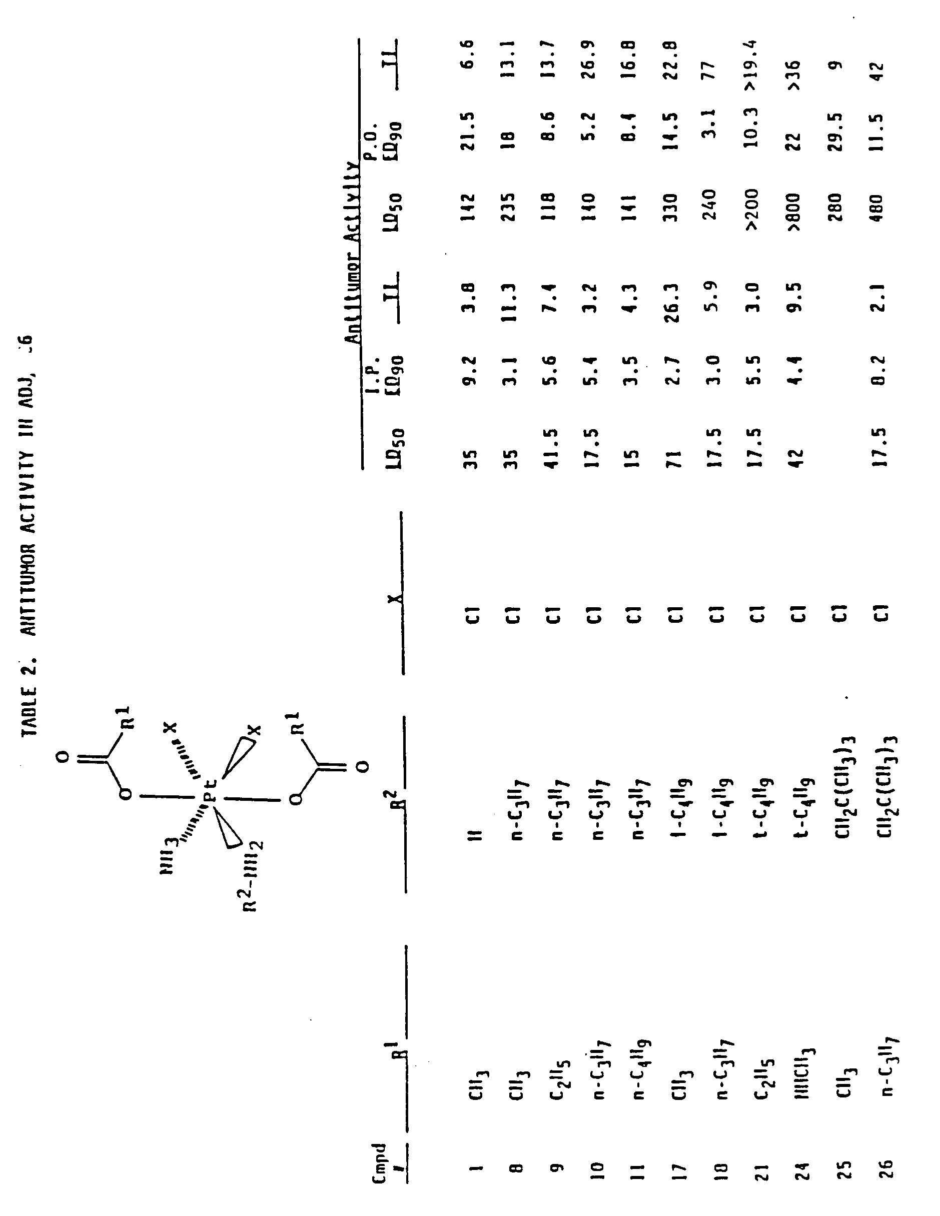

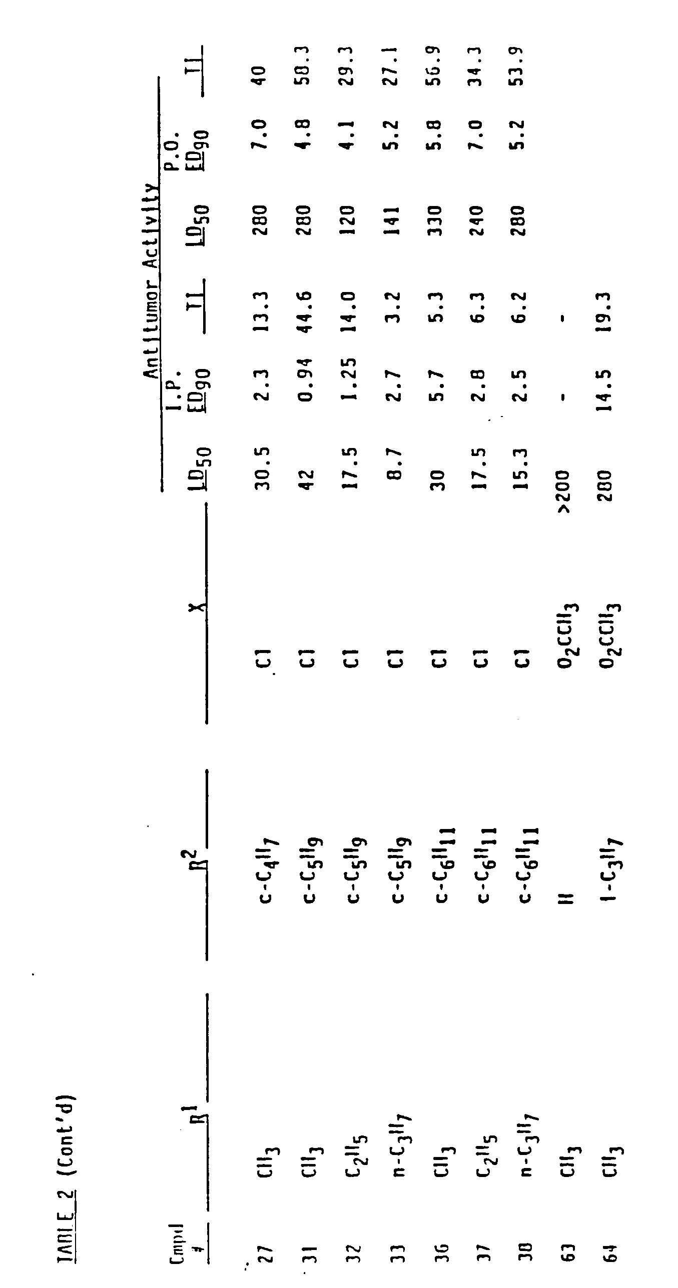

The T.I. values indicate useful activity for the complexes, particularly for oral administration where the T.I. values were significantly higher than for parenteral (I.P.) administration.

## Claims (Claims for the following Contracting State(s): AT, BE, CH, DE, FR, GB, GR, IT, LI, LU, NL, SE)

1. A Pt (IV) complex of the formula 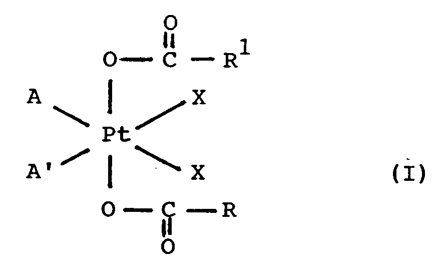 wherein A and A' are independent groups selected from ammine, primary, secondary and tertiary C₁-C₁₀ straight chain, branched chain and cyclic alkylamines; aromatic, heteroaromatic or heterocyclic radicals, and functionalized amines; R and R¹ are hydrogen, C₁-C₁₀ alkyl, alkenyl, aryl, aralkyl, alkylamino or alkoxy; and X is halogen or alkyl monocarboxylate or dicarboxylate.

2. A Pt (IV) complex according to claim 1 wherein X is Cl, A is NH₃, A' is a cycloalkylamine and R and R¹ are C₁-C₇ alkyl.

3. A Pt (IV) complex according to claim 2 wherein A' is cyclopentylamine or cyclohexylamine and R and R¹ are both propyl.

4. A Pt (IV) complex according to claim 1 wherein X is Cl, A is NH₃, A' is a C₁-C₁₀ alkylamine and R and R¹ are straight or branched chain alkyl with 1-10 carbon atoms, cycloalkyl with 3-7 carbon atoms, alkylamine with 1-6 carbon atoms or alkoxy with 1-6 carbon atoms.

5. A Pt (IV) complex according to claim 1 wherein X is Cl, A and A' are both alkylamine with 1 to 10 carbon atoms and R and R¹ are alkyl with 1 to 10 carbon atoms.

6. A Pt (IV) complex according to claim 1 wherein X is a monocarboxylate or a chelating dicarboxylate, R and R¹ are both C₁ - C₆ alkyl, A is NH₃ and A' is C₁ - C₁₀ alkylamino.

7. A pharmaceutical composition suitable for oral or parenteral administration comprising as the active ingredient an effective amount of a complex according to any of claims 1 to 6.

## Claims (Claims for the following Contracting State(s): ES)

1. A method for the preparation of a Pt (IV) complex of the formula 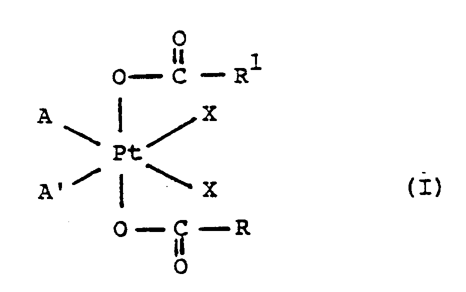 wherein A and A' are independent groups selected from ammine, primary, secondary and tertiary C₁-C₁₀ straight chain, branched chain and cyclic alkylamines; aromatic, heteroaromatic or heterocyclic radicals, and functionalized amines; R and R¹ are hydrogen, C₁-C₁₀ alkyl, alkenyl, aryl, aralkyl, alkylamino or alkoxy; and X is halogen, the method comprising reacting a compound of formula Pt(OH)₂X₂AA' with an appropriate anhydride, isocyanate or pyrocarbonate.

2. A method according to claim 1 wherein X is Cl, A is NH₃, A' is a cycloalkylamine and R and R¹ are C₁-C₇ alkyl.

3. A method according to claim 2 wherein A' is cyclopentylamine or cyclohexylamine and R and R¹ are both propyl.

4. A method according to claim 1 wherein X is Cl, A is NH₃, A' is a C₁-C₁₀ alkylamine and R and R¹ are straight or branched chain alkyl with 1-10 carbon atoms, cycloalkyl with 3-7 carbon atoms, alkylamine with 1-6 carbon atoms or alkoxy with 1-6 carbon atoms.

5. A method complex according to claim 1 wherein X is Cl, A and A' are both alkylamine with 1 to 10 carbon atoms and R and R¹ are alkyl with 1 to 10 carbon atoms.

6. A method for the preparation of a Pt(IV) complex of the formula 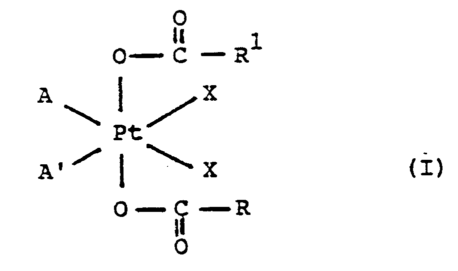 wherein A and A' are independent groups selected from ammine, primary, secondary and tertiary C₁-C₁₀ straight chain, branched chain and cyclic alkylamines; aromatic, heteroaromatic or heterocyclic radicals, and functionalized amines; R and R¹ are hydrogen, C₁-C₁₀ alkyl, alkenyl, aryl, aralkyl, alkylamino or alkoxy; and X is alkyl monocarboxylate or dicarboxylate, the method comprising acylation of the intermediate formed by hydrogen peroxide oxidation of [Pt(H₂O)₂(R¹NH₂)(R²)²⁺] prepared in turn from PtI₂(R¹NH₂)(R²) and silver nitrate.

7. A method according to claim 6 wherein X is a monocarboxylate or a chelating dicarboxylate, R and R¹ are both C₁ - C₆ alkyl, A is NH₃ and A' is C₁ - C₁₀ alkylamino.

8. A method for the preparation of a Pt(IV) complex of the formula 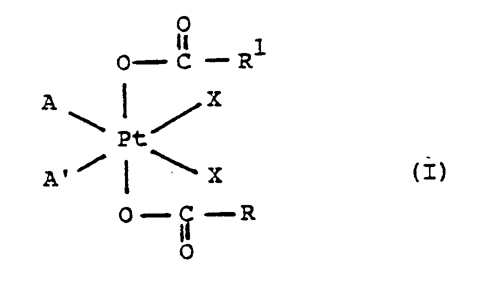 wherein A and A' are NH₃, R and R¹ are CH₃ and the X groups are CBDCA (cyclobutane-1,1-dicarboxylate), the method comprising acylation of the intermediate compound Pt(CBDCA)(OH)₂(NH₃)₂ formed by hydrogen peroxide oxidation of Pt(CBDCA)(NH₃)₂.

## Patentansprüche (Patentansprüche für folgende(n) Vertragsstaat(en): AT, BE, CH, DE, FR, GB, GR, IT, LI, LU, NL, SE)

1. Pt(IV)-Komplex der Formel 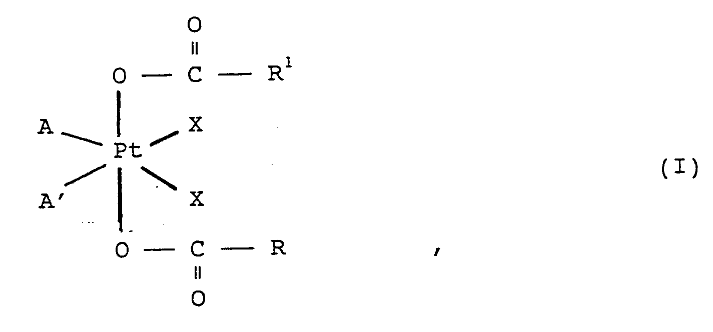 in der A und A' unabhängige Gruppen sind ausgewählt aus Ammin, primären, sekundären und tertiären, geradkettigen, verzweigtkettigen und cyclischen C₁-C₁₀-Alkylaminen, aromatischen, heteroaromatischen oder heterocyclischen Resten und funktionalisierten Aminen, R und R¹ Wasserstoff, C₁-C₁₀-Alkyl, Alkenyl, Aryl, Aralkyl, Alkylamino oder Alkoxy sind und X Halogen, Alkylmonocarboxylat oder -dicarboxylat ist.

2. Pt(IV)Komplex nach Anspruch 1, bei dem X Cl ist, A NH₃ ist, A' ein Cycloalkylamin ist und R und R¹ C₁-C₇-Alkyl sind.

3. Pt(I)-Komplex nach Anspruch 2, bei dem A' Cyclopentylamin oder Cyclohexylamin ist und R und R¹ beide Propyl sind.

4. Pt(IV)-Komplex nach Anspruch 1, bei dem X Cl ist, A NH₃ ist, A' ein C₁-C₁₀-Alkylamin ist und R und R¹ geradkettiges oder verzweigtkettiges Alkyl mit 1 bis 10 Kohlenstoffatomen, Cycloalkyl mit 3 bis 7 Kohlenstoffatomen, Alkylamin mit 1 bis 6 Kohlenstoffatomen oder Alkoxy mit 1 bis 6 Kohlenstoffatomen sind.

5. Pt(IV)-Komplex nach Anspruch 1, bei dem X Cl ist, A und A' beide Alkylamin mit 1 bis 10 Kohlenstoffatomen sind und R und R¹ Alkyl mit 1 bis 10 Kohlenstoffatomen sind.

6. Pt(IV)-Komplex nach Anspruch 1, bei dem X ein Monocarboxylat oder ein chelatisierendes Dicarboxylat ist, R und R¹ beide C₁-C₆-Alkyl sind, A NH₃ ist und A' C₁-C₁₀-Alkylamin ist.

7. Pharmazeutische Zusammensetzung, die zur oralen oder parenteralen Verabreichung geeignet ist und als aktiven Bestandteil eine wirksame Menge eines Komplexes gemäß einem der Ansprüche 1 bis 6 umfaßt.

## Patentansprüche (Patentansprüche für folgende(n) Vertragsstaat(en): ES)

1. Verfahren zur Herstellung eines Pt(IV)-Komplexes der Formel 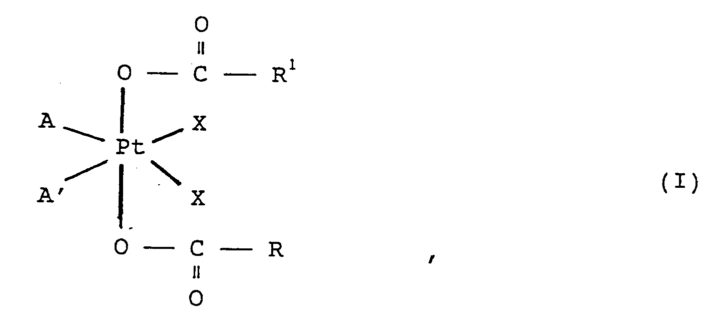 in der A und A' unabhängige Gruppen sind ausgewählt aus Ammin, primären, sekundären und tertiären, geradkettigen, verzweigtkettigen und cyclischen C₁-C₁₀-Alkylaminen, aromatischen, heteroaromatischen oder heterocyclischen Resten und funktionalisierten Aminen, R und R¹ Wasserstoff, C₁-C₁₀-Alkyl, Alkenyl, Aryl, Aralkyl, Alkylamino oder Alkoxy sind und X Halogen ist, bei dem eine Verbindung der Formel Pt(OH)₂X₂AA' mit einem geeigneten Anhydrid, Isocyanat oder Pyrocarbonat umgesetzt wird.

2. Verfahren nach Anspruch 1, bei dem X Cl ist, A NH₃ ist, A' ein Cycloalkylamin ist und R und R¹ C₁-C₇-Alkyl sind.

3. Verfahren nach Anspruch 2, bei dem A' Cyclopentylamin oder Cyclohexylamin ist und R und R¹ beide Propyl sind.

4. Verfahren nach Anspruch 1, bei dem X Cl ist, A NH₃ ist, A' ein C₁-C₁₀-Alkylamin ist und R und R¹ geradkettiges oder verzweigtkettiges Alkyl mit 1 bis 10 Kohlenstoffatomen, Cycloalkyl mit 3 bis 7 Kohlenstoffatomen, Alkylamin mit 1 bis 6 Kohlenstoffatomen oder Alkoxy mit 1 bis 6 Kohlenstoffatomen sind.

5. Verfahren nach Anspruch 1, bei dem X Cl ist, A und A' beide Alkylamin mit 1 bis 10 Kohlenstoffatomen sind und R und R¹ Alkyl mit 1 bis 10 Kohlenstoffatomen sind.

6. Verfahren zur Herstellung eines Pt(IV)-Komplexes der Formel 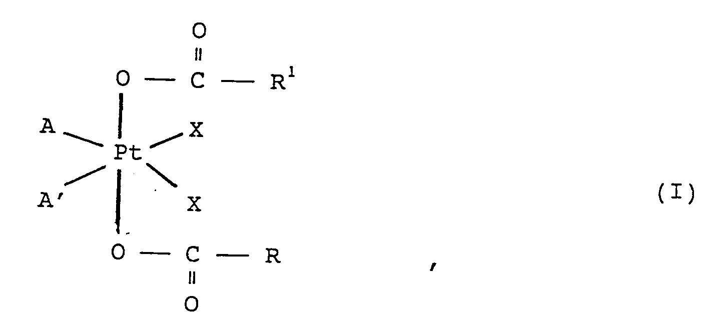 in der A und A' unabhängige Gruppen sind ausgewählt aus Ammin, primären, sekundären und tertiären, geradkettigen, verzweigtkettigen und cyclischen C₁-C₁₀-Alkylaminen, aromatischen, heteroaromatischen oder heterocyclischen Resten und funktionalisierten Aminen, R und R¹ Wasserstoff, C₁-C₁₀-Alkyl, Alkenyl, Aryl, Aralkyl, Alkylamino oder Alkoxy sind und X Alkylmonocarboxylat oder -dicarboxylat ist, wobei das Verfahren eine Acylierung des Zwischenprodukts umfaßt, das durch Wasserstoffperoxidoxiation von [Pt(H₂O)₂(R¹NH₂)(R²)²⁺] worden ist, das wiederum aus PtI₂(R¹NH₂)(R²) und Sibernitrat hergestellt worden ist.

7. Verfahren nach Anspruch 1, bei dem X ein Monocarboxylat oder ein chelatisierendes Dicarboxylat ist, R und R¹ beide C₁-C₆-Alkyl sind, A NH₃ ist und A' C₁-C₁₀-Alkylamino ist.

8. Verfahren zur Herstellung eines Pt(IV)-Komplexes der Formel 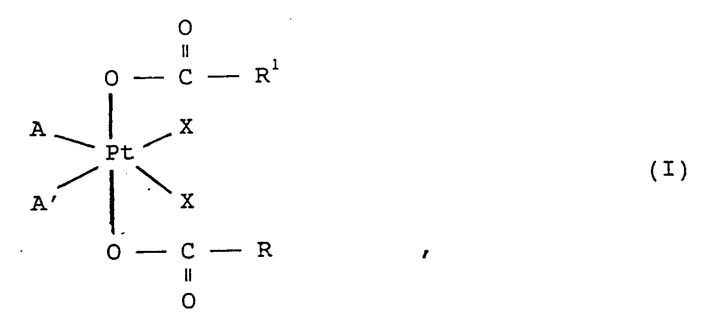 in der A und A' NH₃ sind, R und R¹ CH₃ und die X-Gruppen CBDCA (Cyclobutan-1,1-dicarboxylat) sind, wobei das Verfahren eine Acylierung der Zwischenproduktverbindung Pt(CBDCA)(OH)₂(NH₃)₂ umfaßt, die durch Wasserstoffperoxidoxiation von Pt(CBDCA)(NH₃)₂ gebildet worden ist.

## Revendications (Revendications pour l'(les) Etat(s) contractant(s) suivant(s): AT, BE, CH, DE, FR, GB, GR, IT, LI, LU, NL, SE)

1. Complexe de Pt (IV), de formule 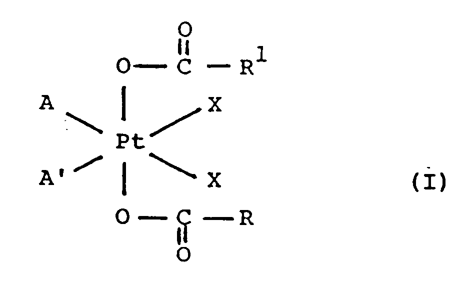 dans laquelle A et A' représentent des groupes indépendants, choisis parmi les amines et alkylamines en C₁-C₁₀, primaires, secondaires ou tertiaires, à chaîne linéraire, ramifiée ou cyclique ; les radicaux aromatiques, hétéroaromatiques ou hétérocycliques, et les amines portant d'autres fonctions ; R et R¹ représentent un atome d'hydrogène, un groupe alkyle en C₁-C₁₀, alcényle, aryle, aralkyle, alkylamino ou alcoxy ; et X représente un atome d'halogène ou un monocarboxylate ou dicarboxylate d'alkyle.

2. Complexe de Pt (IV) selon la revendication 1, dans lequel X est Cl, A est NH₃, A' est une cycloalkylamine et R et R¹ représentent un groupe alkyle en C₁-C₇.

3. Complexe de Pt (IV) selon la revendication 2, dans lequel A' est la cyclopentylamine ou la cyclohexylamine et R et R¹ représentent tous deux un groupe propyle.

4. Complexe de Pt (IV) selon la revendication 1, dans lequel X est Cl, A est NH₃, A' est une alkylamine en C₁-C₁₀ et R et R¹ représentent un groupe alkyle à chaîne linéaire ou ramifiée, ayant 1-10 atomes de carbone, cycloalkyle ayant 3-7 atomes de carbone, alkylamine ayant 1-6 atomes de carbone ou alcoxy ayant 1-6 atomes de carbone.

5. Complexe de Pt (IV) selon la revendication 1, dans lequel X est Cl, A et A' sont tous deux une alkylamine ayant 1-10 atomes de carbone et R et R¹ représentent un groupe alkyle ayant 1 à 10 atomes de carbone.

6. Complexe de Pt (IV) selon la revendication 1, dans lequel X est un monocarboxylate ou un dicarboxylate chélatant, R et R¹ représentent tous deux un groupe alkyle en C₁-C₆, A est NH₃ et A' est un groupe alkylamino en C₁-C₁₀.

7. Composition pharmaceutique appropriée pour l'administration par voie orale ou parentérale, comprenant, comme ingrédient actif, une quantité efficace d'un complexe selon l'une quelconque des revendications 1 à 6.

## Revendications (Revendications pour l'(les) Etat(s) contractant(s) suivant(s): ES)

1. Procédé de préparation d'un complexe de Pt (IV), de formule 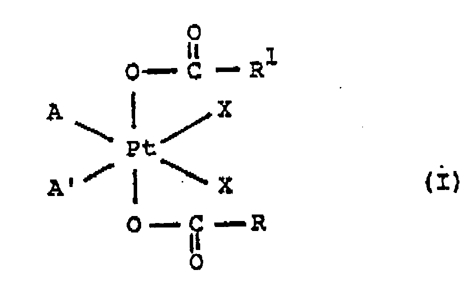 dans laquelle A et A' représentent des groupes indépendants, choisis parmi les amines et alkylamines en C₁-C₁₀, primaires, secondaires et tertiaires, à chaîne linéaire, ramifiée ou cyclique les radicaux aromatiques, hétéroaromatiques ou hétérocycliques, et les amines portant d'autres fonctions ; R et R¹ représentent un atome d'hydrogène, un groupe alkyle en C₁-C₁₀, alcényle, aryle, aralkyle, alkylamino ou alcoxy ; et X représente un atome d'halogène, le procédé comprenant la réaction d'un composé de formule Pt(OH)₂X₂AA' avec un anhydride, isocyanate ou pyrocarbonate approprié.

2. Procédé selon la revendication 1, dans lequel X est Cl, A est NH₃, A' est une cycloalkylamine et R et R¹ représentent un groupe alkyle en C₁-C₇.

3. Procédé selon la revendication 2, dans lequel A' est la cyclopentylamine ou la cyclohexylamine et R et R¹ représentent tous deux un groupe propyle.

4. Procédé selon la revendication 1, dans lequel X est Cl, A est NH₃, A' est une alkylamine en C₁-C₁₀ et R et R¹ représentent un groupe alkyle à chaîne linéaire ou ramifiée, ayant 1-10 atomes de carbone, cycloalkyle ayant 3-7 atomes de carbone, alkylamine ayant 1-6 atomes de carbone ou alcoxy ayant 1-6 atomes de carbone.

5. Procédé selon la revendication 1, dans lequel X est Cl, A et A' sont tous deux une alkylamine ayant 1-10 atomes de carbone et R et R¹ représentent un groupe alkyle ayant 1 à 10 atomes de carbone.

6. Procédé de préparation d'un complexe de Pt (IV), de formule 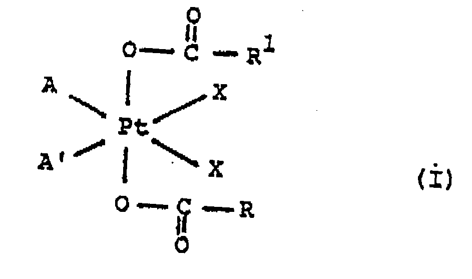 dans laquelle A et A' représentent des groupes indépendants, choisis parmi les amines et alkylamines en C₁-C₁₀, primaires, secondaires et tertiaires, à chaîne linéaire, ramifiée ou cyclique ; les radicaux aromatiques, hétéroaromatiques ou hétérocycliques, et les amines portant d'autres fonctions ; R et R¹ représentent un hydrogène, un groupe alkyle en C₁-C₁₀, alcényle, aryle, aralkyle, alkylamino ou alcoxy ; et X représente un monocarboxylate ou dicarboxylate d'alkyle, le procédé comprenant l'acylation du produit intermédiaire formé par oxydation au peroxyde d'hydrogène de [Pt(H₂O)₂(R¹NH₂)(R²)²⁺], qui a été obtenu à son tour à partir de PtI₂(R¹NH₂)(R²) et de nitrate d'argent.

7. Procédé selon la revendication 6, dans lequel X est un monocarboxylate ou un dicarboxylate chélatant, R et R¹ représentent tous deux un groupe alkyle en C₁-C₆, A est NH₃ et A' est un groupe alkylamino en C₁-C₁₀.

8. Procédé de préparation d'un complexe de Pt (IV), de formule 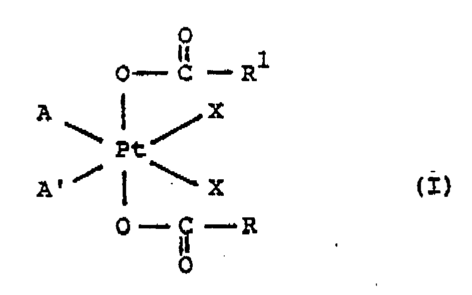 dans laquelle A et A' représentent NH₃, R et R¹ représentent CH₃ et les groupes X représentent le CBDCA (cyclobutane-1,1-dicarboxylate), le procédé comprenant l'acylation du composé intermédiaire Pt(CBDCA)(OH)₂(NH₃)₂, formé par oxydation au peroxyde d'hydrogène de Pt(CBDCA)(NH₃)₂.
